# EUROPEAN PATENT SPECIFICATION

(11) **EP 3 342 348 B1**
(45) Date of publication and mention of the grant of the patent: **11.03.2020**
(21) Application number: 17197772.1
(22) Date of filing: 23.10.2017
(51) Int. Cl.: A61B 6/03, A61B 6/00

(54) **MEDICAL DEVICE**
MEDIZINISCHE VORRICHTUNG
DISPOSITIF MÉDICAL

(30) Priority: 29.12.2016 KR 20160182914
(43) Date of publication of application: 04.07.2018
(73) Proprietor: Samsung Electronics Co., Ltd., Suwon-si, Gyeonggi-do (KR)
(72) Inventor: Dave, Vikram Keertikumar, Suwon-si, Gyeonggi-do (KR)
(74) Representative: Gulde & Partner

(56) References cited:
- WO-A1-2016/172611
- WO-A2-2014/013367
- DE-A1-102013 209 250
- US-B1- 6 431 751

## Description

### BACKGROUND

The present disclosure relates to a medical device, and more particularly, to a medical device that is convertible to have mobility.

An x-ray imaging apparatus is an apparatus that irradiates an object with x-rays and analyzes x-rays that have been transmitted through the object to recognize an inner structure of the object. Since x-ray transmittance varies according to a tissue forming an object, an inner structure of the object may be imaged using an attenuation coefficient which is a numerical value of x-ray transmittance.

X-ray imaging apparatuses can be classified as a simple x-ray imaging device that transmits x-rays in one direction and a computed tomography (CT) device that transmits x-rays in various directions and reconstructs an image with a computer. The CT device is referred to as a computer tomography device or a computerized tomography device.

The CT device can be classified as a fixed CT device and a mobile CT device depending on mobility. The mobile CT device is a device that is devised so that a state of an object can be imaged in real time and is getting the limelight nowadays due to being able to image an object even during an operation and being able to freely move in an operating room.

Because both the fixed CT device and the mobile CT device have advantages and disadvantages, being equipped with both the fixed CT device and the mobile CT device may be preferable from a user's point of view. However, being equipped with both of the two types of CT devices may be a large financial burden on the user. Also, when volumes of the fixed CT device and the mobile CT device are taken into consideration, being equipped with both of the types of CT devices may be difficult due to a spatial problem.

A medical device comprising a first main body and a second mobile main body, and a C-arm adapted to be movably installed on one of the first or second main body, is known from US patent 6,431,751 B1. The first main body is mounted to the ceiling, and the second main body is a mobile cart. The C-arm can be transferred from one main body to the other, and electrically connected selectively to each of them.

### SUMMARY

To address the above-discussed deficiencies, it is a primary object to provide a medical device that has an improved structure to be freely convertible between a fixed type and a mobile type.

Additional aspects of the disclosure will be set forth in part in the description which follows and, in part, will be obvious from the description, or may be learned by practice of the disclosure.

According to an aspect of the present disclosure, a medical device includes a first main body, a second main body having mobility and arranged to be adjacent to the first main body, and a gantry movably installed on the first main body and the second main body to be electrically connected to the first main body or electrically connected to the second body.

According to an aspect of the present disclosure, the medical device may further include a mobile platform arranged below the second main body to give mobility to the second main body, and the second main body may be movably installed on the mobile platform.

The mobile platform may include a platform body having a plurality of casters installed at a lower surface and a platform leg rotatably coupled to the platform body.

The gantry electrically connected to the second body may integrally move with the second main body, and the medical device further includes a guide rail installed on the mobile platform to guide movement of the second main body. The guide rail includes a first guide rail installed on the platform body and a second guide rail installed on the platform leg to be separably coupled to the first guide rail.

The first main body and the second main body may be arranged to be disposed on a straight line in a first direction, and the platform leg may be provided to be rotatable between a first position disposed on a straight line with the platform body in the first direction and a second position disposed on a straight line with the platform body in a second direction which is perpendicular to the first direction.

According to an aspect of the present disclosure, the medical device may further include a belt installed at the mobile platform to be adjacent to the guide rail to guide movement of the second main body together with the guide rail.

According to an aspect of the present disclosure, the medical device may further include a belt movement involving module installed inside the second main body to be involved in movement of the belt, and the belt movement involving module may include a pulley unit that includes a plurality of idler pulleys and a drive pulley disposed above the plurality of idler pulleys.

The belt movement involving module may further include a tension adjusting unit coupled to any one of the plurality of idler pulleys to adjust tension of the belt that changes with rotation of the platform leg.

According to an aspect of the present disclosure, the medical device may further include a locking member arranged between the second main body and the mobile platform to selectively lock the second main body to the mobile platform.

A plurality of support members may be installed at a lower surface of the mobile platform, the plurality of casters may be involved in movement of the mobile platform, and the plurality of support members may be involved in fixation of the mobile platform.

The plurality of casters may be installed at the platform body to selectively protrude from the platform body.

The gantry may include a gantry body having a bore formed therein and a gantry protruding portion protruding from the gantry body and having an adapter installed therein.

The first main body may be arranged outside the gantry, a first guide may be recessed and formed at an inner surface of the first main body facing the gantry to guide movement of the gantry by corresponding to the gantry protruding portion, and a first adapter that is electrically connected to the adapter may be installed at the first guide.

The second main body may be arranged outside the gantry, a second guide may be recessed and formed at an inner surface of the second main body facing the gantry to guide movement of the gantry by corresponding to the gantry protruding portion, and a second adapter that is electrically connected to the adapter may be installed at the second guide.

According to another aspect of the present disclosure, a medical device includes a gantry provided so that imaging an object is possible, a main body arranged outside the gantry and having a first guide rail, configured to guide movement of the gantry, formed at an inner surface of the main body facing the gantry, and a mobile platform arranged below a portion of the main body and having a second guide rail, configured to guide movement of the portion of the main body, formed therein.

The main body may include a first main body and a second main body arranged above the mobile platform to be movable along the second guide rail and separably coupled to the first main body.

The first main body may include a first adapter, the second main body may include a second adapter, and the gantry may include an adapter selectively electrically connected to the first adapter or the second adapter as the gantry moves along the first guide rail.

The mobile platform may include a platform body having a plurality of casters installed at a lower surface of the platform body and a platform leg foldably coupled to the platform body.

According to still another aspect of the present disclosure, a medical device includes a gantry provided so that imaging an object is possible and a main body having a guide, configured to guide linear movement of the gantry, formed therein and having a plurality of adapters that are electrically connectable to the gantry as the gantry moves.

Before undertaking the DETAILED DESCRIPTION below, it may be advantageous to set forth definitions of certain words and phrases used throughout this patent document: the terms "include" and "comprise," as well as derivatives thereof, mean inclusion without limitation; the term "or," is inclusive, meaning and/or; the phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like; and the term "controller" means any device, system or part thereof that controls at least one operation, such a device may be implemented in hardware, firmware or software, or some combination of at least two of the same. It should be noted that the functionality associated with any particular controller may be centralized or distributed, whether locally or remotely. Definitions for certain words and phrases are provided throughout this patent document, those of ordinary skill in the art should understand that in many, if not most instances, such definitions apply to prior, as well as future uses of such defined words and phrases.

### BRIEF DESCRIPTION OF THE DRAWINGS

For a more complete understanding of the present disclosure and its advantages, reference is now made to the following description taken in conjunction with the accompanying drawings, in which like reference numerals represent like parts:
FIG. 1 illustrates an overall configuration diagram of a medical device according to an embodiment of the present disclosure;
FIGS. 2A and 2B illustrate a process of moving a gantry in the medical device according to an embodiment of the present disclosure;
FIG. 3 illustrates the process of moving the gantry in the medical device according to an embodiment of the present disclosure in a different angle from FIGS. 2A and 2B.
FIG. 4 illustrates a state in which the gantry is electrically connected to a second main body in the medical device according to an embodiment of the present disclosure;
FIG. 5 illustrates an enlarged view of a mobility implementation structure of the second main body in the medical device according to an embodiment of the present disclosure;
FIG. 6 illustrates an enlarged view of an adapter and a first adapter in the medical device according to an embodiment of the present disclosure;
FIG. 7 illustrates a mobile module in a state in which a platform leg is folded in the medical device according to an embodiment of the present disclosure;
FIG. 8 illustrates the mobile module in the state in which the platform leg is folded in the medical device according to an embodiment of the present disclosure in a different angle from FIG. 7;
FIG. 9 illustrates the mobile module in a state in which the platform leg is unfolded in the medical device according to an embodiment of the present disclosure;
FIGS. 10A and 10B illustrate a process of coupling a first guide rail and a second guide rail after the platform leg is unfolded in the medical device according to an embodiment of the present disclosure;
FIG. 11 illustrates a belt movement involving module in the medical device according to an embodiment of the present disclosure; and
FIGS. 12A and 12B illustrate operation states of a tension adjusting unit in the medical device according to an embodiment of the present disclosure.

### DETAILED DESCRIPTION

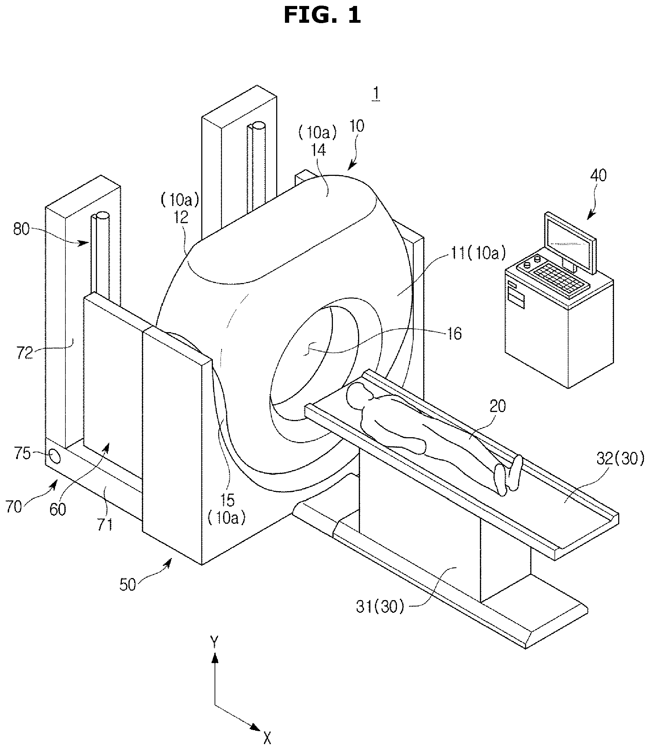

FIGURES 1 through 12B, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged system or device.

A medical device of the present disclosure is a device for imaging an object and obtaining information on a biological tissue. The medical device of the present disclosure may include an x-ray imaging device for obtaining an image of an inside of an object using x-rays and a magnetic resonance imaging device for obtaining an image of an inside of an object using the magnetic resonance phenomenon. Here, the x-ray imaging device may be classified depending on an object to be imaged as a general x-ray imaging device, a dental x-ray imaging device, a mammography device for mammography, and the like, and may be classified depending on a imaging angle as a general x-ray imaging device that performs imaging at a single imaging angle during one-time imaging and a tomography device that performs imaging at various imaging angles during one-time imaging and combines images into a single image. The medical device of the present disclosure can be any device capable of imaging an object and obtaining information of the object, and the type of the medical device is not limited to the above examples. Hereinafter, description will be mainly given on a computed tomography (CT) device as an example of the medical device of the present disclosure.

An object may be a living body of a human being or an animal but is not limited thereto, and anything whose internal structure can be imaged using the medical device of the present disclosure can be an object. Hereinafter, description will be mainly given on the human body as an example of the object.

FIG. 1 illustrates an overall configuration diagram of a medical device according to an embodiment of the present disclosure. Hereinafter, "X" represents a first direction, and "Y" represents a second direction. The reference numeral "75" indicates a rotating shaft.

As illustrated in FIG. 1, a medical device 1 may include a gantry 10. An x-ray source that generates x-rays and irradiates an object 20 with x-rays and an x-ray detector that detects x-rays that transmitted through the object 20 and acquires x-ray data may be disposed inside the gantry 10.

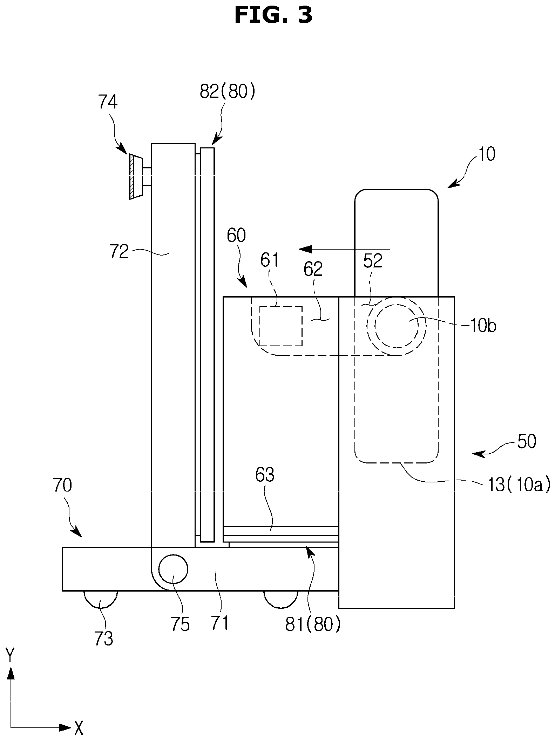

The gantry 10 may include a gantry body 10a. The gantry body 10a may include a front surface 11 facing the object 20, a rear surface 12 corresponding to the front surface 11, a lower surface 13 (see FIG. 3) facing a surface at which the medical device 1 is installed (hereinafter, a bottom surface), an upper surface 14 corresponding to the lower surface 13, and a side surface 15 connecting the front surface 11, the rear surface 12, the lower surface 13, and the upper surface 14.

The gantry 10 may further include a bore 16. The bore 16 may be formed in the gantry body 10a. The object 20 may be inserted into the bore 16. Imaging of the object 20 can be performed while the object 20 is inserted into the bore 16.

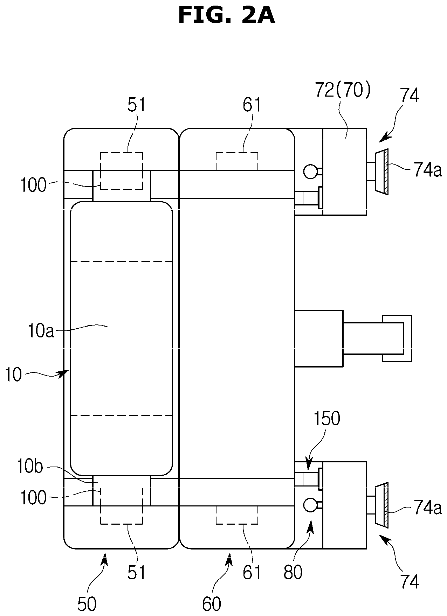

The gantry 10 may further include a gantry protruding portion 10b (see FIG. 2A). The gantry protruding portion 10b may be formed to protrude from the gantry body 10a. An adapter 100 (see FIG. 2A) may be installed at the gantry protruding portion 10b.

The adapter 100 may be selectively coupled to a first adapter 51 (see FIG. 2A) installed in a first main body 50 and a second adapter 61 (see FIG. 2A) installed in a second main body 60. The gantry 10 may receive power supply from the first main body 50 or transmit and receive an electrical signal to and from the first main body 50 by electrical coupling between the adapter 100 and the first adapter 51. Also, the gantry 10 may receive power supply from the second main body 60 or transmit and receive an electrical signal to and from the second main body 60 by electrical coupling between the adapter 100 and the second adapter 61.

The gantry 10 may be movably formed. Specifically, the gantry 10 may be movably installed on the first main body 50 and the second main body 60.

The medical device 1 may further include a main body arranged outside the gantry 10. Guides 52 and 62 configured to guide linear movement of the gantry 10 may be formed in the main body. Also, the plurality of adapters 51 and 61 that can be electrically connected to the gantry 10 as the gantry 10 moves may be provided in the main body. The main body may include the first main body 50 forming a fixed module together with the gantry 10. Also, the main body may include the second main body 60 forming a mobile module together with the gantry 10. The first main body 50 and the second main body 60 may be arranged to be adjacent to each other. Preferably, the first main body 50 may be arranged to face a table 30. In other words, the first main body 50 may be arranged between the table 30 and the second main body 60. However, an arrangement relationship between the first main body 50 and the second main body 60 may be changed in various ways and is not limited to the above example.

The medical device 1 may further include a mobile platform 70 arranged below the second main body 60 to give mobility to the mobile module. The mobile platform 70 may include a platform body 71 having a plurality of casters 73 (see FIG. 3) installed at a lower surface. The plurality of casters 73 may be installed at the platform body 71 to selectively protrude from the platform body 71. Also, the mobile platform 70 may further include a platform leg 72 foldably coupled to the platform body 71. From another aspect, the platform leg 72 may be rotatably coupled to the platform body 71.

The mobile module may be movably installed on the mobile platform 70. That is, the second main body 60 may be installed on the mobile platform 70 to integrally move with the gantry 10 disposed on the second main body 60.

The medical device 1 may further include a guide rail 80 installed on the mobile platform 70 to guide movement of the mobile module. In other words, the guide rail 80 may be installed on the mobile platform 70 to guide movement of the second main body 60 that moves on the mobile platform 70. The guide rail 80 may longitudinally extend on the platform body 71 and the platform leg 72. Preferably, the guide rail 80 may longitudinally extend discontinuously on the platform body 71 and the platform leg 72. The guide rail 80 may include a first guide rail 81 installed on the platform body 71. The guide rail 80 may further include a second guide rail 82 installed on the platform leg 72 to be separably coupled to the first guide rail 81.

The medical device 1 may further include the table 30 provided so that the object 20 is located thereon. The table 30 may include a fixed portion 31 and a cradle 32. The cradle 32 may be disposed above the fixed portion 31. Specifically, the cradle 32 may be slidably disposed above the fixed portion 31. The cradle 32 may be provided to be inserted into the bore 16 together with the object 20. The cradle 32 may be formed with a material through which x-rays can pass so that 3D stereoscopic imaging of the object 20 located on the cradle 32 is possible. The table 30 may be anything on which the object 20 may be located, and the type of the table 30 is not limited to the above example. As an example, the table 30 may include an operating table, a fixed table, a mobile table, and the like.

The table 30 may be provided as a separate configuration from the gantry 10.

The medical device 1 may further include a work station 40 configured to display an image of the object 20 and receive a control command related to an overall operation of the medical device 1 from a user.

In this way, because the medical device 1 of the present disclosure can implement both a medical device having a fixed module and a medical device having a mobile module using a single gantry 10, convenience in use of the user can be considerably improved. Also, because the medical device 1 of the present disclosure can implement both the medical device 1 having a fixed module and a medical device having a mobile module using a single gantry 10, a financial burden on the user can be considerably reduced. Also, when the medical device 1 of the present disclosure is used, a limitation in terms of installation space can be lower compared to the case in which a medical device having a fixed module and a medical device having a mobile module are separately provided. Also, because the medical device 1 of the present disclosure is easily convertible between a medical device having a fixed module and a medical device having a mobile module, convenience in use of the user can be maximized.

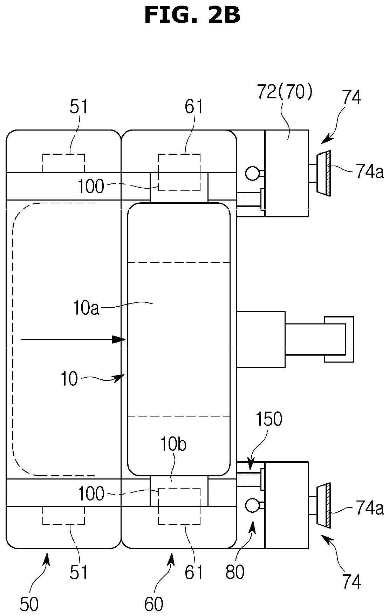

FIGS. 2A and 2B illustrate a process of moving a gantry in the medical device according to an embodiment of the present disclosure, and FIG. 3 illustrates the process of moving the gantry in the medical device according to an embodiment of the present disclosure in a different angle from FIGS. 2A and 2B. FIG. 2A illustrates a case in which the gantry 10 is located on the first main body 50, and FIG. 2B illustrates a case in which the gantry 10 is located on the second main body 60.

As illustrated in FIGS. 2A to 3, the gantry 10 may be movably installed on the first main body 50 and the second main body 60. The gantry 10 may be movably installed on the first main body 50 and the second main body 60 to be capable of linear movement.

The gantry 10 may be electrically connected to the first main body 50 or electrically connected to the second main body 60. That is, when the gantry 10 is located on the first main body 50, the gantry 10 may be electrically connected to the first main body 50 and implement a fixed module. Also, when the gantry 10 is located on the second main body 60, the gantry 10 may be electrically connected to the second main body 60 and implement a mobile module. An electrical connection between the gantry 10 and the first main body 50 and an electrical connection between the gantry 10 and the second main body 60 do not interfere with each other.

The first main body 50 and the second main body 60 may be arranged to be located on a straight line in the first direction X.

The first main body 50 and the second main body 60 may be separably coupled to each other. The medical device 1 may further include a guide formed in the main body to guide movement of the gantry 10. Specifically, the guide may be longitudinally recessed and formed in the first direction X at an inner surface of the main body facing the gantry 10.

The guide may include the first guide 52 formed in the first main body 50. The first guide 52 may be recessed and formed at an inner surface of the first main body 50 facing the gantry 10 to guide movement of the gantry 10 by corresponding to the gantry protruding portion 10b. The first adapter 51 may be installed in the first guide 52.

The guide may further include the second guide 62 formed in the second main body 60. The second guide 62 may be recessed and formed at an inner surface of the second main body 60 facing the gantry 10 to guide movement of the gantry 10 by corresponding to the gantry protruding portion 10b. The second adapter 61 may be installed in the second guide 62.

The medical device 1 may further include a belt 150 installed to guide movement of the mobile module together with the guide rail 80. That is, the belt 150 may be installed to guide movement of the second main body 60 together with the guide rail 80. The belt 150 may be installed at the mobile platform 70 to be adjacent to the guide rail 80. The belt 150 and the guide rail 80 may be arranged to be spaced a predetermined distance from each other so as not to interfere with each other. The belt 150 may be installed to be located inside the guide rail 80. Both ends of the belt 150 may be fixed to the mobile platform 70. Specifically, one end of the belt 150 may be fixed to the platform body 71. The other end of the belt 150 may be fixed to the platform leg 72.

The belt 150 may include a serrated belt.

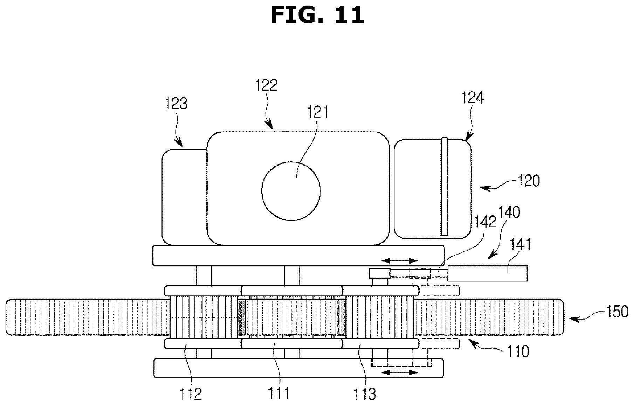

The medical device 1 may further include a belt movement involving module (see FIG. 11) provided to be involved in movement of the belt 150. The belt movement involving module may be installed inside the second main body 60.

The belt movement involving module may include a pulley unit 110.

The belt movement involving module may further include a tension adjusting unit 140.

The belt movement involving module will be described in detail below.

The medical device 1 may further include a locking member 90 provided to selectively lock the mobile module to the mobile platform 70. That is, the locking member 90 may be provided to selectively lock the second main body 60 to the mobile platform 70. The locking member 90 may be arranged between the second main body 60 and the mobile platform 70. Preferably, the locking member 90 may be installed on the mobile platform 70 to be separable from the second main body 60. However, the position of the locking member 90 is not limited to the above example and may be changed in various ways. For example, the locking member 90 may be installed at the second main body 60 to be separable from the mobile platform 70. The locking member 90 restricts movement of the second main body 60 by coupling the second main body 60 to the mobile platform 70. The second main body 60 is movable only when the locking state by the locking member 90 is released.

The medical device 1 may further include a plurality of support members 74. The plurality of support members 74 may be installed at the lower surface of the mobile platform 70. The plurality of support members 74 serve to fix the mobile platform 70. The plurality of support members 74 may include a slide preventer 74a to prevent the mobile platform 70 from sliding on the bottom surface. The slide preventer 74a may be formed at the plurality of support members 74 to be able to come into contact with the bottom surface and may have an elastic material. As an example, the slide preventer 74a may be formed of rubber, silicone, urethane, and the like.

The plurality of casters 73 may be involved in movement of the mobile platform 70. The plurality of support members 74 may be involved in fixation of the mobile platform 70. From another aspect, the plurality of casters 73 and the plurality of support members 74 may selectively come into contact with the bottom surface. The plurality of casters 73 may come into contact with the bottom surface when the mobile platform 70 moves. Conversely, the plurality of support members 74 may come into contact with the bottom surface when the mobile platform 70 is fixed.

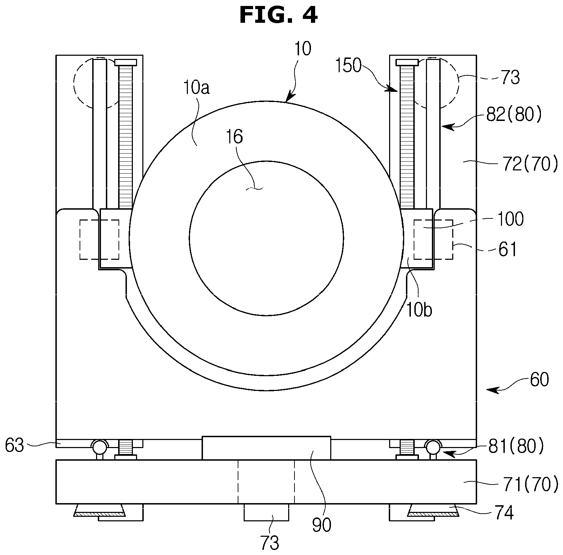

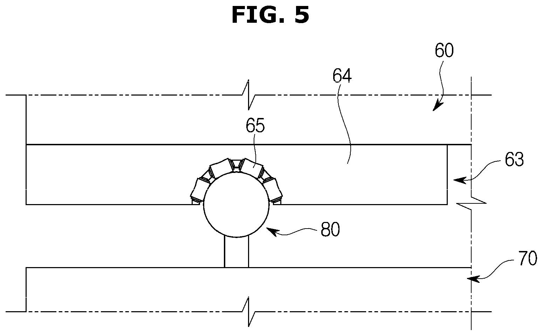

FIG. 4 illustrates a state in which the gantry is electrically connected to a second main body in the medical device according to an embodiment of the present disclosure, and FIG. 5 illustrates an enlarged view of a mobility implementation structure of the second main body in the medical device according to an embodiment of the present disclosure. For reference, FIG. 4 illustrates a state in which the second main body 60 is fixed to the mobile platform 70 by the locking member 90. Here, movement of the second main body 60 is restricted by the locking member 90.

As illustrated in FIGS. 4 and 5, the second main body 60 may be installed on the mobile platform 70 to be movable along the guide rail 80.

The second main body 60 may include a slider 63. The slider 63 may be formed at one surface of the second main body 60 facing the guide rail 80. The slider 63 may include a slider body 64 and a plurality of rollers 65 coupled to the slider body 64 to be able to come into contact with the guide rail 80. As another embodiment, the slider 63 may include the slider body 64 and a plurality of balls coupled to the slider body 64 to be able to come into contact with the guide rail 80.

The slider 63 may be movably coupled to the guide rail 80. In other words, the slider 63 may move along the guide rail 80 while being coupled to the guide rail 80.

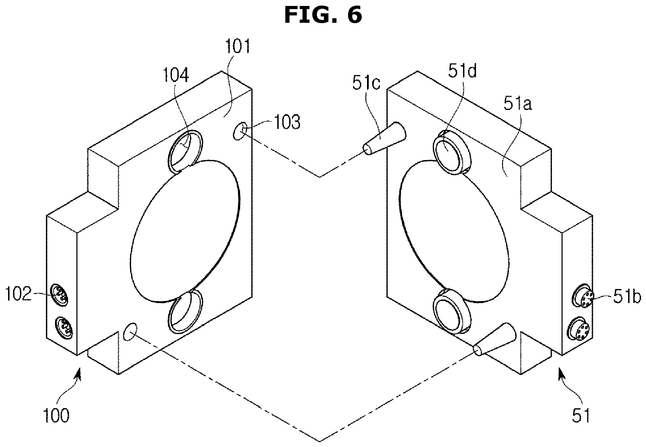

FIG. 6 illustrates an enlarged view of an adapter and a first adapter in the medical device according to an embodiment of the present disclosure.

As illustrated in FIG. 6, the gantry 10 may include the adapter 100.

The first main body 50 may include the first adapter 51 electrically coupled to the adapter 100 when the gantry 10 is located on the first main body 50.

The second main body 60 may include the second adapter 61 electrically coupled to the adapter 100 when the gantry 10 is located on the second main body 60.

Because the shapes of the first adapter 51 and the second adapter 61 are equal to each other, description will be mainly given on the first adapter 51 below.

The adapter 100 may include an adapter body 101 installed in the gantry 10. Specifically, the adapter body 101 may be installed at the gantry protruding portion 10b.

The adapter 100 may further include one or more connectors 102 provided at one side of the adapter body 101. Various electronic components arranged inside the gantry 10 may be electrically connected to the one or more connectors 102. As an example, various electronic components arranged inside the gantry 10 may be electrically connected to the one or more connectors 102 via wires (not illustrated).

The adapter 100 may further include one or more guide holes 103 formed in the adapter body 101 to correspond to one or more guide pins 51c. The number and the shape of the one or more guide holes 103 may correspond to the number and the shape of the one or more guide pins 51c. The one or more guide pins 51c and the one or more guide holes 103 assist the adapter 100 and the first adapter 51 to be coupled to each other at accurate positions.

The adapter 100 may further include one or more coupling holes 104 formed at the adapter body 101 to correspond to one or more coupling pins 51d. The number and the shape of the one or more coupling holes 104 may correspond to the number and the shape of the one or more coupling pins 51d. The adapter 100 and the first adapter 51 may be electrically connected to each other by coupling between the one or more coupling pins 51d and the one or more coupling holes 104.

The first adapter 51 may include a first adapter body 51a installed in the first main body 50. Specifically, the first adapter body 51a may be installed in the first guide 52 of the first main body 50.

The first adapter 51 may further include one or more first connectors 51b provided at one side of the first adapter body 51a. Various electronic components arranged inside the first main body 50 may be electrically connected to the one or more first connectors 51b. As an example, various electronic components arranged inside the first main body 50 may be electrically connected to the one or more first connectors 51b via wires (not illustrated).

The first adapter 51 may further include the one or more guide pins 51c formed in the first adapter body 51a. The one or more guide pins 51c may be coupled to the one or more guide holes 103. The one or more guide pins 51c may be separably coupled to the one or more guide holes 103. Specifically, the one or more guide pins 51c may be selectively coupled to the one or more guide holes 103 by an actuator. Here, the actuator may be operated by an electric motor, a hydraulic pressure, a pneumatic pressure, and the like. Also, the one or more guide pins 51c may be selectively coupled to the one or more guide holes 103 by an electrical signal.

The first adapter 51 may further include the one or more coupling pins 51d formed in the first adapter body 51a. The one or more coupling pins 51d may be electrically coupled to the one or more coupling holes 104. The one or more coupling pins 51d may be separably electrically coupled to the one or more coupling holes 104. Specifically, the one or more coupling pins 51d may be selectively electrically coupled to the one or more coupling holes 104 by an actuator. Here, the actuator may be operated by an electric motor, a hydraulic pressure, a pneumatic pressure, and the like. Also, the one or more coupling pins 51d may be selectively electrically coupled to the one or more coupling holes 104 by an electrical signal.

In this way, the gantry 10 may receive power supply from the first main body 50 or transmit and receive an electrical signal to and from the electronic components arranged inside the first main body 50 by electrical coupling between the adapter 100 and the first adapter 51. Here, the electrical signal may include a control signal.

The electrical coupling between the gantry 10 and the first main body 50 or the second main body 60 is not limited to the above examples and may be realized using various methods.

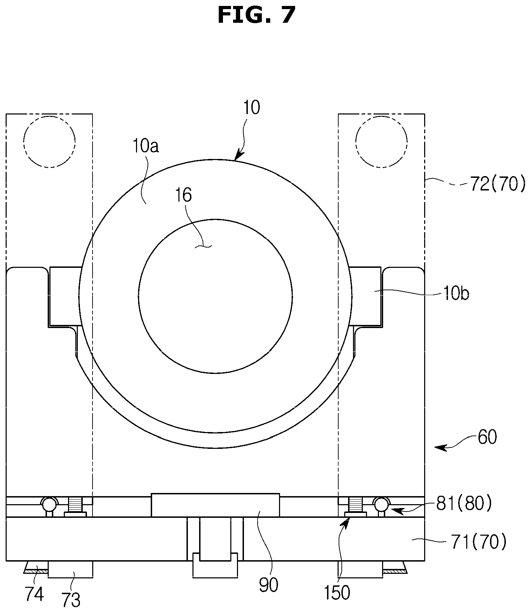

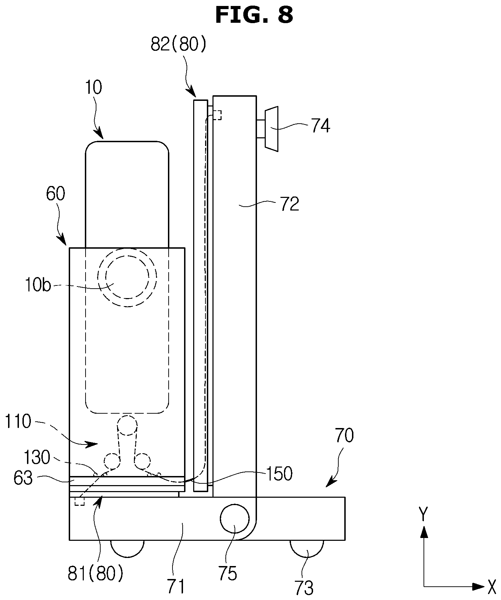

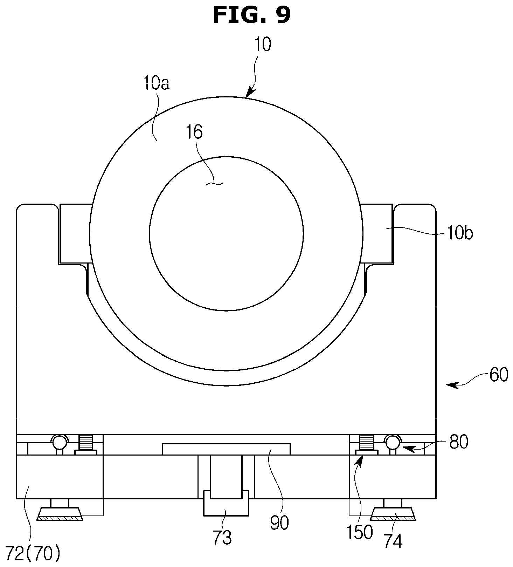

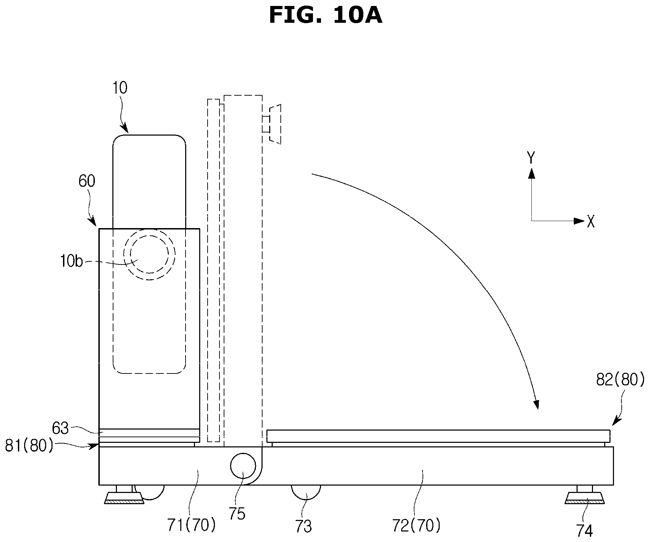

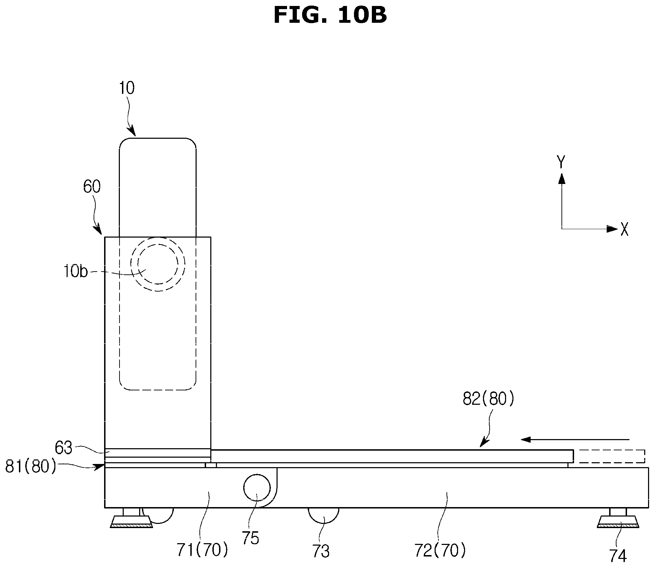

FIG. 7 illustrates a mobile module in a state in which a platform leg is folded in the medical device according to an embodiment of the present disclosure, and FIG. 8 illustrates the mobile module in the state in which the platform leg is folded in the medical device according to an embodiment of the present disclosure in a different angle from FIG. 7. FIG. 9 illustrates the mobile module in a state in which the platform leg is unfolded in the medical device according to an embodiment of the present disclosure, and FIGS. 10A and 10B illustrate a process of coupling a first guide rail and a second guide rail after the platform leg is unfolded in the medical device according to an embodiment of the present disclosure.

As illustrated in FIGS. 7 to 10B, the platform leg 72 of the mobile platform 70 may be foldably installed. In other words, the platform leg 72 may be installed at the platform body 71 to be rotatable about the rotating shaft 75.

The platform leg 72 may be provided to be rotatable between a first position and a second position. The platform leg 72 may be located on a straight line with the platform body 71 in the first direction X at the first position. Also, the platform leg 72 may be located on a straight line with the platform body 71 in the second direction Y at the second position. The first direction X and the second direction Y may be in perpendicular relationship with each other.

When the platform leg 72 is located at the first position, the guide rail 80 and the guides 52 and 62 may be parallel to each other.

The second main body 60 may be present while being fixed to the mobile platform 70 by the locking member 90. The gantry 10 that is moved from the first main body 50 to the second main body 60 may be fixed to the second main body 60. As an example, the gantry 10 may be coupled to the second main body 60 by coupling between the adapter 100 and the second adapter 61. That is, the adapter 100 and the second adapter 61 may serve to physically couple the gantry 10 and the second main body 60 to each other as well as electrically connect electronic components inside the gantry 10 and electronic components inside the second main body 60 to one another. In this way, the gantry 10, the second main body 60, and the mobile platform 70 may be coupled to each other to be integrally movable. Here, the platform leg 72 of the mobile platform 70 may be located at the second position. That is, the platform leg 72 may be present in a folded state. For convenience, the gantry 10, the second main body 60, and the mobile platform 70 being coupled to each other to be integrally movable is referred to as a "mobile module assembly." The mobile module assembly is movable by the casters 73 while the platform leg 72 is folded. Here, the casters 73 may protrude from the platform body 71 and come into contact with the bottom surface.

Upon reaching a desired position, the mobile module assembly may be fixed to the bottom surface for imaging the object. When the mobile module assembly reaches a desired position, the platform leg 72 which is folded is unfolded, and the one or more support members 74 may come into contact with the bottom surface. That is, the platform leg 72 may be located at the first position. Here, the casters 73 that give mobility to the mobile module assembly may be lifted toward inside of the platform body 71 to be spaced apart from the bottom surface. When the platform leg 72 is unfolded in the first direction X, the first guide rail 81 and the second guide rail 82 are present while being spaced apart from each other as illustrated in FIG. 10A. When the guide rail 80 is discontinuously present as illustrated in FIG. 10A, because it is difficult to expect smooth movement of the mobile module, the second guide rail 82 is pressed and coupled to the first guide rail 81 as illustrated in FIG. 10b. When the first guide rail 81 and the second guide rail 82 are coupled and a guide rail 80 is formed, a preparation process for moving the mobile module is finished. When the locking state by the locking member 90 is released, the mobile module may freely move along the guide rail 80. The user may perform imaging of the object while moving the mobile module.

When the mobile module assembly moves, the belt 150 may be present in a folded state as the platform leg 72. When the mobile module moves for imaging the object, the belt 150 may be present in an unfolded state as the platform leg 72.

The pulley unit 110 illustrated in FIG. 8 will be described in detail below.

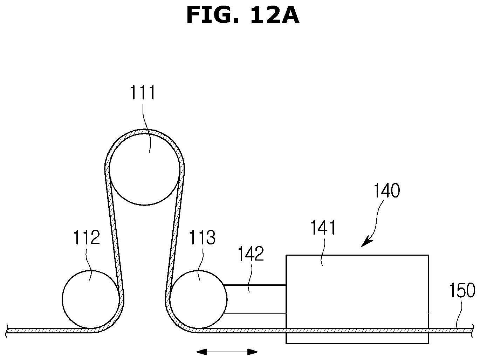

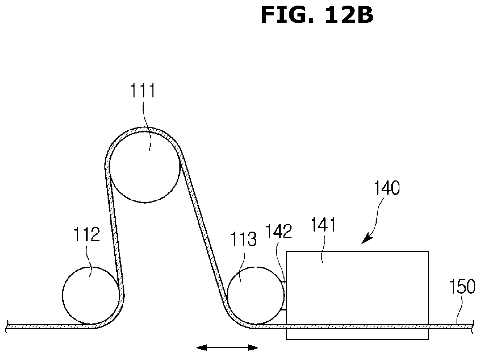

FIG. 11 illustrates a belt movement involving module in the medical device according to an embodiment of the present disclosure, and FIGS. 12A and 12B illustrate operation states of a tension adjusting unit in the medical device according to an embodiment of the present disclosure.

As illustrated in FIGS. 11 to 12B, the medical device 1 may further include a belt movement involving module installed inside the second main body 60 to be involved in movement of the belt 150.

The belt movement involving module may include the pulley unit 110. The pulley unit 110 may include a plurality of idler pulleys 112 and 113 and a drive pulley 111 disposed above the plurality of idler pulleys 112 and 113. Preferably, the pulley unit 110 may include the two idler pulleys 112 and 113 and the drive pulley 111. The plurality of idler pulleys 112 and 113 may include a first idler pulley 112 and a second idler pulley 113 coupled to the tension adjusting unit 140. Specifically, the second idler pulley 113 may be coupled to a mover 142 of the tension adjusting unit 140 and integrally move with the mover 142.

The belt movement involving module may further include a pulley unit driver 120. The pulley unit driver 120 may include a motor (not illustrated) configured to generate rotational force of a rotating shaft 121. The pulley unit driver 120 may further include the rotating shaft 121 configured to connect the motor and the drive pulley 111 to each other. The pulley unit driver 120 may further include a gearbox 122 in which various gears for transmitting power required to rotate the rotating shaft 121 are embedded. The pulley unit driver 120 may further include an encoder 123, a brake 124, and the like.

The belt movement involving module may further include a plurality of guide rollers 130 (see FIG. 8) configured to guide movement of the belt 150.

The belt movement involving module may further include the tension adjusting unit 140. The tension adjusting unit 140 may be coupled to any one of the plurality of idler pulleys 112 and 113 to adjust tension of the belt 150 that changes with rotation of the platform leg 72. The tension adjusting unit 140 may include a driver 141 and a mover 142 coupled to the driver 141 to be capable of linear reciprocating movement. The mover 142 of the tension adjusting unit 140 may be coupled to the second idler pulley 113.

FIG. 12A illustrates a state of the belt movement involving module when the platform leg 72 is located at the second position, and FIG. 12B shows a state of the belt movement involving module when the platform leg 72 is located at the first position.

When the platform leg 72 is located at the first position, considerable tension may act on the belt 150. To prevent tension of a predetermined level or higher from acting on the belt 150 like this, the tension adjusting unit 140 may move the mover 142 to which the second idler pulley 113 is coupled so that a distance between the first idler pulley 112 and the second idler pulley 113 increases.

When the platform leg 72 is located at the second position, the belt 150 may be relatively loose. To prevent the belt 150 from loosening to a predetermined extent or more like this, the tension adjusting unit 140 may move the mover 142 to which the second idler pulley 113 is coupled so that the distance between the first idler pulley 112 and the second idler pulley 113 decreases.

Because both a fixed medical device and a mobile medical device can be implemented using a single gantry, a financial burden on a user can be reduced, and a limitation in terms of installation space can be lower compared to the case in which a fixed medical device and a mobile medical device are separately provided.

Because conversion between the fixed medical device and the mobile medical device is easy, convenience in use of the user can be improved.

Although the present disclosure has been described with an exemplary embodiment, various changes and modifications may be suggested to one skilled in the art. It is intended that the present disclosure encompass such changes and modifications as long as they fall within the scope of the appended claims.

## Claims

1. A medical device (1) comprising:
a first main body (50);
a second main body (60), the second main body (60) is configured to be mobile and arranged to be adjacent to the first main body (50); and
a gantry (10) adapted to be movably installed either on the first main body (50) or on the second main body (60),
wherein each of the first main body (50) and the second main body (60) comprises a guide to guide linear movement of the gantry (10) from one main body to the other,
wherein the gantry (10) is configured to be electrically connected to the first main body (50) when being installed on the first main body (50),
wherein the gantry (10) is configured to be electrically connected to the second main body (60) when being installed on the second main body (60).

2. The medical device of claim 1, further comprising a mobile platform (70) arranged below the second main body, the mobile platform is configured to provide mobility to the second main body,
wherein the second main body is movably installed on the mobile platform.

3. The medical device of claim 2, further comprising a locking member (90) arranged between the second main body and the mobile platform, the locking member is configured to selectively lock the second main body to the mobile platform.

4. The medical device of claim 2, wherein the mobile platform includes:
a platform body (71) having a plurality of casters (73) installed at a lower surface; and
a platform leg (72) rotatably coupled to the platform body.

5. The medical device of claim 4, wherein:
when the gantry is electrically connected to the second body, the gantry integrally moves with the second main body; and
the medical device further includes a guide rail (80) installed on the mobile platform, the guide rail is configured to guide movement of the second main body.

6. The medical device of claim 5, wherein the guide rail includes:
a first guide rail (81) installed on the platform body; and
a second guide rail (82) installed on the platform leg, the second guide rail is separably coupled to the first guide rail.

7. The medical device of claim 5, further comprising a belt (150) installed at the mobile platform to be adjacent to the guide rail, the belt is configured to guide movement of the second main body together with the guide rail.

8. The medical device of claim 7, further comprising a belt movement involving module installed inside the second main body , the belt movement involving module is configured to move the belt,
wherein the belt movement involving module includes a pulley unit (110) that includes a plurality of idler pulleys (112, 113) and a drive pulley (111) disposed above the plurality of idler pulleys.

9. The medical device of claim 8, further comprising a tension adjusting unit (140) coupled to at least one of the plurality of idler pulleys, the tension adjusting unit is configured to adjust tension of the belt that changes with rotation of the platform leg.

10. The medical device of claim 4, wherein:
the first main body and the second main body are arranged to be disposed on a straight line in a first direction; and
the platform leg is configured to be rotatable between a first position disposed on a straight line with the platform body in the first direction and a second position disposed on a straight line with the platform body in a second direction which is perpendicular to the first direction.

11. The medical device of claim 4, wherein:
a plurality of support members (74) are installed at a lower surface of the mobile platform;
the plurality of casters are configured to move the mobile platform; and
the plurality of support members are configured to affix the mobile platform.

12. The medical device of claim 4, wherein the plurality of casters are installed at the platform body to selectively protrude from the platform body.

13. The medical device of claim 1, wherein the gantry includes:
a gantry body (10a) having a bore (16) formed therein; and
a gantry protruding portion (10b) protruding from the gantry body and having an adapter (100) installed therein.

14. The medical device of claim 13, wherein:
the first main body is arranged outside the gantry;
a first guide (52) is recessed and formed at an inner surface of the first main body facing the gantry to guide movement of the gantry by corresponding to the gantry protruding portion; and
a first adapter (51) that is electrically connected to the adapter, the first adapter is installed at the first guide.

15. The medical device of claim 14, wherein:
the second main body is arranged outside the gantry;
a second guide (62) is recessed and formed at an inner surface of the second main body facing the gantry to guide movement of the gantry by corresponding to the gantry protruding portion; and
a second adapter (61) that is electrically connected to the adapter, the second adapter is installed at the second guide.

## Patentansprüche

1. Medizinische Vorrichtung (1) umfassend:
einen ersten Grundkörper (50);
einen zweiten Grundkörper (60), wobei der zweite Grundkörper (60) dazu ausgestaltet ist, mobil zu sein, und derart angeordnet ist, dass er an den ersten Grundkörper (50) angrenzt; und
eine Gantry (10), die dafür geeignet ist, beweglich entweder an dem ersten Grundkörper (50) oder an dem zweiten Grundkörper (60) installiert zu sein,
wobei jeder von dem ersten Grundkörper (50) und dem zweiten Grundkörper (60) eine Führung umfasst, um eine lineare Bewegung der Gantry (10) von einem Grundkörper zum anderen zu führen,
wobei die Gantry (10) dazu ausgestaltet ist, elektrisch mit dem ersten Grundkörper (50) verbunden zu sein, wenn sie an dem ersten Grundkörper (50) installiert ist,
wobei die Gantry (10) dazu ausgestaltet ist, elektrisch mit dem zweiten Grundkörper (60) verbunden zu sein, wenn sie an dem zweiten Grundkörper (60) installiert ist.

2. Medizinische Vorrichtung nach Anspruch 1, ferner umfassend eine mobile Plattform (70), die unter dem zweiten Grundkörper angeordnet ist, wobei die mobile Plattform dazu ausgestaltet ist, dem zweiten Grundkörper Mobilität zu verleihen,
wobei der zweite Grundkörper beweglich an der mobilen Plattform installiert ist.

3. Medizinische Vorrichtung nach Anspruch 2, ferner umfassend ein Fixierelement (90), das zwischen dem zweiten Grundkörper und der mobilen Plattform angeordnet ist, wobei das Fixierelement dazu ausgestaltet ist, den zweiten Grundkörper selektiv an der mobilen Plattform zu fixieren.

4. Medizinische Vorrichtung nach Anspruch 2, wobei die mobile Plattform Folgendes enthält:
einen Plattformkörper (71) mit einer Mehrzahl von Rollen (73), die an einer unteren Fläche installiert sind; und
einen Plattformschenkel (72), der drehbar mit dem Plattformkörper gekoppelt ist.

5. Medizinische Vorrichtung nach Anspruch 4, wobei:
wenn die Gantry elektrisch mit dem zweiten Körper verbunden ist, die Gantry sich einstückig mit dem zweiten Grundkörper bewegt; und
die medizinische Vorrichtung ferner eine Führungsschiene (80) enthält, die an der mobilen Plattform installiert ist, wobei die Führungsschiene dazu ausgestaltet ist, die Bewegung des zweiten Grundkörpers zu führen.

6. Medizinische Vorrichtung nach Anspruch 5, wobei die Führungsschiene Folgendes enthält:
eine erste Führungsschiene (81), die an dem Plattformkörper installiert ist; und
eine zweite Führungsschiene (82), die an dem Plattformschenkel installiert ist, wobei die zweite Führungsschiene trennbar mit der ersten Führungsschiene gekoppelt ist.

7. Medizinische Vorrichtung nach Anspruch 5, ferner umfassend einen Riemen (150), der an der mobilen Plattform derart installiert ist, dass er an die Führungsschiene angrenzt, wobei der Riemen dazu ausgestaltet ist, die Bewegung des zweiten Grundkörpers zusammen mit der Führungsschiene zu führen.

8. Medizinische Vorrichtung nach Anspruch 7, ferner umfassend ein eine Riemenbewegung verursachendes Modul, das innerhalb des zweiten Grundkörpers installiert ist, wobei das die Riemenbewegung verursachende Modul dazu ausgestaltet ist, den Riemen zu bewegen,
wobei das die Riemenbewegung verursachende Modul eine Rolleneinheit (110) enthält, die eine Mehrzahl von Umlenkrollen (112, 113) und eine Antriebsrolle (111) enthält, die über der Mehrzahl von Umlenkrollen angeordnet ist.

9. Medizinische Vorrichtung nach Anspruch 8, ferner umfassend eine Spannungseinstelleinheit (140), die mit mindestens einer aus der Mehrzahl von Umlenkrollen gekoppelt ist, wobei die Spannungseinstelleinheit dazu ausgestaltet ist, die Spannung des Riemens einzustellen, die sich mit der Drehung der Plattformschenkel verändert.

10. Medizinische Vorrichtung nach Anspruch 4, wobei:
der erste Grundkörper und der zweite Grundkörper derart angebracht sind, dass sie auf einer geraden Linie in einer ersten Richtung angeordnet sind; und
der Plattformschenkel dazu ausgestaltet ist, zwischen einer ersten Position, die auf einer geraden Linie mit dem Plattformkörper in der ersten Richtung angeordnet ist, und einer zweiten Position, die auf einer geraden Linie mit dem Plattformkörper in einer zweiten Richtung angeordnet ist, die senkrecht zu der ersten Richtung ist, drehbar zu sein.

11. Medizinische Vorrichtung nach Anspruch 4, wobei:
eine Mehrzahl von Stützelementen (74) an einer unteren Fläche der mobilen Plattform installiert ist;
die Mehrzahl von Rollen dazu ausgestaltet ist, die mobile Plattform zu bewegen; und
die Mehrzahl von Stützelementen dazu ausgestaltet ist, die mobile Plattform zu befestigen.

12. Medizinische Vorrichtung nach Anspruch 4, wobei the Mehrzahl von Rollen an dem Plattformkörper derart installiert sind, dass sie selektiv von dem Plattformkörper vorstehen.

13. Medizinische Vorrichtung nach Anspruch 1, wobei die Gantry Folgendes enthält:
einen Gantry-Körper (10a), der eine darin ausgebildete Bohrung (16) enthält; und
einen Gantry-Vorsprungsab schnitt (10b), der von dem Gantry-Körper vorsteht und einen darin installierten Adapter (100) enthält.

14. Medizinische Vorrichtung nach Anspruch 13, wobei:
der erste Grundkörper außerhalb der Gantry angeordnet ist;
eine erste Führung (52) ausgespart ist und an einer inneren Fläche des ersten Grundkörpers, die der Gantry zugewandt ist, ausgebildet ist, um die Bewegung der Gantry zu führen, indem sie dem Gantry-Vorsprungsabschnitt entspricht; und
ein erster Adapter (51), der elektrisch mit dem Adapter verbunden ist, wobei der erste Adapter an der ersten Führung installiert ist.

15. Medizinische Vorrichtung nach Anspruch 14, wobei:
der zweite Grundkörper außerhalb der Gantry angeordnet ist;
eine zweite Führung (62) ausgespart ist und an einer inneren Fläche des zweiten Grundkörpers, die der Gantry zugewandt ist, ausgebildet ist, um die Bewegung der Gantry zu führen, indem sie dem Gantry-Vorsprungsabschnitt entspricht; und
ein zweiter Adapter (61), der elektrisch mit dem Adapter verbunden ist, wobei der zweite Adapter an der zweiten Führung installiert ist.

## Revendications

1. Dispositif médical (1), comprenant :
un premier corps principal (50) ;
un deuxième corps principal (60), le deuxième corps principal (60) étant configuré pour être mobile et agencé pour être adjacent au premier corps principal (50) ; et
un portique (10) conçu pour être installé de manière mobile sur le premier corps principal (50) ou sur le deuxième corps principal (60),
le premier corps principal (50) et le deuxième corps principal (60) comprenant chacun un guide pour guider un mouvement linéaire du portique (10) depuis un corps principal vers l'autre,
le portique (10) étant configuré pour être électriquement connecté au premier corps principal (50) lorsqu'il est installé sur le premier corps principal (50),
le portique (10) étant configuré pour être électriquement connecté au deuxième corps principal (60) lorsqu'il est installé sur le deuxième corps principal (60).

2. Dispositif médical selon la revendication 1, comprenant en outre une plateforme mobile (70) agencée sous le deuxième corps principal, la plateforme mobile étant configurée pour conférer une mobilité au deuxième corps principal,
le deuxième corps principal étant installé de manière mobile sur la plateforme mobile.

3. Dispositif médical selon la revendication 2, comprenant en outre un élément de verrouillage (90) agencé entre le deuxième corps principal et la plateforme mobile, l'élément de verrouillage étant configuré pour verrouiller sélectivement le deuxième corps principal à la plateforme mobile.

4. Dispositif médical selon la revendication 2, dans lequel la plateforme mobile comprend :
un corps de plateforme (71) comprenant une pluralité de roulettes (73) installées au niveau d'une surface inférieure ; et
un montant de plateforme (72) couplé en rotation au corps de plateforme.

5. Dispositif médical selon la revendication 4, dans lequel :
lorsque le portique est connecté électriquement au deuxième corps, le portique se déplace intégralement avec le deuxième corps principal ; et
le dispositif médical comprend en outre un rail de guidage (80) installé sur la plateforme mobile, le rail de guidage étant configuré pour guider un mouvement du deuxième corps principal.

6. Dispositif médical selon la revendication 5, dans lequel le rail de guidage comprend :
un premier rail de guidage (81) installé sur le corps de plateforme ; et
un deuxième rail de guidage (82) installé sur le montant de plateforme, le deuxième rail de guidage étant couplé séparément au premier rail de guidage.

7. Dispositif médical selon la revendication 5, comprenant en outre une courroie (150) installée au niveau de la plateforme mobile pour être adjacente au rail de guidage, la courroie étant configurée pour guider un mouvement du deuxième corps principal conjointement avec le rail de guidage.

8. Dispositif médical selon la revendication 7, comprenant en outre un module impliqué dans le mouvement de la courroie installé à l'intérieur du deuxième corps principal, le module impliqué dans le mouvement de la courroie étant configuré pour déplacer la courroie,
le module impliqué dans le mouvement de la courroie comprenant une unité de poulie (110) qui comprend une pluralité de poulies libres (112, 113) et une poulie d'entraînement (111) disposée au-dessus de la pluralité de poulies libres.

9. Dispositif médical selon la revendication 8, comprenant en outre une unité d'ajustement de la tension (140) couplée à au moins une poulie libre parmi la pluralité de poulies libres, l'unité d'ajustement de la tension étant configurée pour ajuster la tension de la courroie qui change avec une rotation du montant de la plateforme.

10. Dispositif médical selon la revendication 4, dans lequel :
le premier corps principal et le deuxième corps principal sont agencés pour être disposés sur une ligne droite dans une première direction ; et
le montant de la plateforme est configuré pour être rotatif entre une première position disposée sur une ligne droite avec le corps de plateforme dans la première direction et une deuxième position disposée sur une ligne droite avec le corps de plateforme dans une deuxième direction qui est perpendiculaire à la première direction.

11. Dispositif médical selon la revendication 4, dans lequel :
une pluralité d'éléments supports (74) sont installés au niveau d'une surface inférieure de la plateforme mobile ;
la pluralité de roulettes sont configurées pour déplacer la plateforme mobile ; et
la pluralité d'éléments supports sont configurés pour fixer la plateforme mobile.

12. Dispositif médical selon la revendication 4, dans lequel la pluralité de roulettes sont installées au niveau du corps de la plateforme pour dépasser sélectivement du corps de la plateforme.

13. Dispositif médical selon la revendication 1, dans lequel le portique comprend :
un corps de portique (10a) dans lequel un alésage (16) est formé ; et
une partie de dépassement du portique (10b) dépassant du corps de portique et dans laquelle un adaptateur (100) est installé.

14. Dispositif médical selon la revendication 13, dans lequel :
le premier corps principal est agencé à l'extérieur du portique ;
un premier guide (52) est en retrait et formé au niveau d'une surface intérieure du premier corps principal orientée vers le portique pour guider un mouvement du portique par correspondance avec la partie de dépassement du portique ; et
un premier adaptateur (51) qui est connecté électriquement à l'adaptateur, le premier adaptateur étant installé au niveau du premier guide.

15. Dispositif médical selon la revendication 14, dans lequel :
le deuxième corps principal est agencé à l'extérieur du portique ;
un deuxième guide (62) est en retrait et formé au niveau d'une surface intérieure du deuxième corps principal orientée vers le portique pour guider un mouvement du portique par correspondance avec la partie de dépassement du portique ; et
un deuxième adaptateur (61) qui est connecté électriquement à l'adaptateur, le deuxième adaptateur étant installé au niveau du deuxième guide.
